# EUROPEAN PATENT SPECIFICATION

(11) **EP 4 126 196 B1**
(45) Date of publication and mention of the grant of the patent: **01.07.2026**
(21) Application number: 20926774.9
(22) Date of filing: 03.08.2020
(51) Int. Cl.: A61N 1/362, A61N 1/365, A61B 5/021, A61B 5/00

(54) **TREATMENT OF DRUG RESISTANT HYPERTENSION**
BEHANDLUNG VON ARZNEIMITTELRESISTENTEM BLUTHOCHDRUCK
TRAITEMENT DE L'HYPERTENSION RÉSISTANTE AUX MÉDICAMENTS

(30) Priority: 27.03.2020 WO PCT/US2020/025447; 05.05.2020 US 202063101544 P
(43) Date of publication of application: 08.02.2023
(73) Proprietor: BaroPace, Inc., Ashland, OR 97520 (US)
(72) Inventor: MICHAEL, Burnam, Ashland, OR 97520 (US); ELI, Gang, Los Angeles, CA 90046 (US)
(74) Representative: Eisenführ Speiser
(86) International application number: PCT/US2020/044784
(87) International publication number: WO 2021/194543

(56) References cited:
- US-A1- 2004 049 235
- US-A1- 2016 175 595
- US-B2- 6 662 047
- US-B2- 7 460 899
- US-B2- 7 660 628
- US-B2- 8 509 893
- US-B2- 9 636 513

## Description

### Background

### Field of the Technology

The invention relates to an apparatus for treating diastolic heart failure and for controlling blood pressure in patients who have proven to be resistant to drug treatments for blood pressure control in the fields characterized by CPC A61N 1/36564; A6 1N *1*/*3682*; A6 1N *1*/*36585*; A61N /*36117*; and A6 IN 1/36571.

### Description of the Prior Art

US 7,660,628 B2 relates to various aspects to provide an implantable device. In various embodiments, the device comprises at least one port, where each port is adapted to connect a lead with an electrode to the device. The device further includes a stimulation platform, including a sensing circuit connected to the at least one port to sense an intrinsic cardiac signal and a stimulation circuit connected to the at least one port via a stimulation channel to deliver a stimulation signal through the stimulation channel to the electrode. The stimulation circuit is adapted to deliver stimulation signals through the stimulation channel for both neural stimulation therapy and CRM therapy. The sensing and stimulation circuits are adapted to perform CRM functions. The device further includes a controller connected to the sensing circuit and the stimulation circuit to control the neural stimulation therapy and the CRM therapy. Other aspects and embodiments are provided herein.

We have previously disclosed a method to treat drug resistant hypertension and heart failure with preserved ejection fraction, aka diastolic heart failure. See, PCX Patent Applications entitled, "Method and Apparatus for Treatment of Drug Resistant Hypertension Associated with Impaired Left Ventricular Function and Bradycardia Using a Cardiac Pacemaker", PCT/US 19/59703, and entitled "An Intelligently, Continuously and Physiologically Controlled Pacemaker and Method of Operation of the Same", PCT/US2020/25447. That method comprises a blood pressure sensor connected via a secure Bluetooth or similar communication connection to an application containing an algorithm (referenced as PressurePace) that processes the blood pressure reading determining the optimal right atrial pacing rate of the pacemaker. The calculated atrial pacing rate is then transmitted to the pacemaker via an encrypted Bluetooth or similar connection to regulate the implanted pacemaker's right atrial pacing rate thereby optimizing blood pressure and treating diastolic heart failure.

It has been previously published by one of us, Dr. Eli Gang, that atrial subthreshold pacing can have an effect on local tissue refractory properties, such as the inhibition of impulse transmission across an accessory pathway in the heart of a patient

In addition, there is ample evidence in the literature that non-propagated high intensity pacing stimuli delivered to the ventricle during refractoriness (i.e., during the R-wave) can enhance contractile properties of the ventricle.

It has been experimentally shown that atrial pacing can promote the secretion of a hormone produced by atrial tissue, namely, atrial natriuretic peptide, which has been shown to lower blood pressure under certain physiologic conditions. " Noll B, Krappe J, Göke B, Maisch B (1989) Influence of pacing mode and rate on peripheral levels of atrial natriuretic peptide (ANP). PACE 12:1763-1768; and Noll B, Krappe J, Göke B, Maisch B (1990) Atrial natriuretic peptide levels reflect hemodynamic changes under pacemaker stimulation, PACE 13:970-975,

However, there are no publications disclosing use of either subthreshold and/or non-propagated supra-threshold pacing algorithms in the right atrium designed to affect blood pressure via interaction with epicardial cardiac ganglia or other neuroendocrine mechanisms.

### Brief Summary

The invention is defined by the independent claim 1. The illustrated embodiments of the invention include an apparatus used to treat drug resistant hypertension and heart failure with a preserved ejection fraction (aka diastolic heart failure). The apparatus includes a blood pressure measuring device, a cardiac pacemaker, and an application module containing two algorithms or software defined modules linking the blood pressure measuring device and the pacemaker. The measuring device, the pacemaker and application module are mutually communicated through encrypted Bluetooth or a similar wireless communication protocol. The first algorithm or module (hereinafter referenced as "PressurePace") calculates the optimal right atrial pacing rate of the cardiac pacemaker to optimally regulate a patient's blood pressure. The second algorithm or module (hereafter, referenced as the :"Stimulus Architecture Algorithm" or "SAA") calculates the optimal stimulus output of the pacemaker's right atrial pacing electrode, namely such parameters as amplitude, pulse width, and frequency of subthreshold stimulations grouped into one bundled stimulus train to be delivered to the right atrial tissue to produce one paced heartbeat. This of right atrial stimulation causes feedback inhibition of cardiac-adjacent sympathetic and/or parasympathetic nervous system tissues, resulting in a lowering of blood pressure.

In summary, the illustrated non-claimed embodiments include a method of right atrial pacing of a heart of a patient. The method includes the steps of: stimulating right atrial tissue of the heart using a right atrial lead of a dual chamber cardiac pacemaker to pace the heart with a stimulus architecture protocol; and stimulating local sympathetic and/or parasympathetic tissues with the stimulus architecture protocol proximate to the paced right atrial tissue causing nervous system activity that inhibits the autonomous nervous system to reduce blood pressure.

The step of stimulating right atrial tissue of the heart using a right atrial lead of a dual chamber cardiac pacemaker to pace the heart with a stimulus architecture protocol includes the step of stimulating right atrial tissue with a selected composite amplitude of the pacing stimulus, a selected number of sub-pulses within the pacing stimulus totaling the composite delivered amplitude, a selected frequency of the sub-pulses, a selected duration of the sub-pulses, and/or a selected refractory period before another impulse can be generated, where the selections are determined by an artificial intelligence derived stimulus architecture protocol.

The step of stimulating right atrial tissue of the heart and stimulating local sympathetic and/or parasympathetic tissues with the stimulus architecture protocol includes the step of stimulating the right atrial tissue of the heart and stimulating local sympathetic and/or parasympathetic tissues using an optimized stimulus protocol devised by artificial intelligence for the patient to reduce blood pressure selected from the group selectively varied stimulus amplitude, selectively varied stimulus duration, selectively varied the ascent and descent phase slopes of stimulation, , selectively varied number of impulses delivered in a train or a succession of closely spaced stimuli, selectively varied frequencies of the impulses delivered as a train or a succession of closely spaced stimuli, and/or selectively varied amplitudes of the individual impulses delivered as a train or a succession of closely spaced stimuli, where intensity of delivered right atrial stimuli may be subthreshold or suprathreshold; and where the impulses may be propagated or nonpropagated, depending on the timing of the impulse, as determined by the stimulus architecture protocol.

The step of stimulating the right atrial tissue of the heart and stimulating local sympathetic and/or parasympathetic tissues using an optimized stimulus protocol devised by artificial intelligence for the patient to reduce blood pressure includes the steps of determining if a cardiac beat is required; and supra-threshold pacing the heart and causing a neuro-inhibitory feedback to lower blood pressure, if a cardiac beat is required.

Alternatively the step of stimulating the right atrial tissue of the heart and stimulating local sympathetic and/or parasympathetic tissues using an optimized stimulus protocol devised by artificial intelligence for the patient to reduce blood pressure includes the steps of: determining if a cardiac beat is required; and sub-threshold pacing the heart to only produce a neuro-inhibitory effect on blood pressure without pacing the heart, if a cardiac beat in not required.

The step of stimulating right atrial tissue of the heart and stimulating local sympathetic and/or parasympathetic tissues with the stimulus architecture protocol includes the step of stimulating the right atrial tissue with a right atrial pacing lead with multiple pacing electrodes such that one pacing electrode comprises a conventional right atrial pacing lead, and one or more other pacing electrodes are arranged and configured to be disposed anatomically proximate to atrial tissue proximate to sympathetic and/or parasympathetic ganglionic tissues for the purpose of stimulating those ganglionic tissues to lower blood pressure.

This disclosure also extends to include a closed loop system. The closed system includes: a blood pressure measuring device for transmitting a blood pressure reading; an applications module receiving the blood pressure reading from the applications module, where the applications module operates under an algorithm that uses the measured blood pressure reading to calculate a selected stimulus architecture of which selectively stimulates the right atrial tissue of the heart and selectively stimulates local sympathetic and/or parasympathetic tissues using an optimized stimulus protocol devised by artificial intelligence for the patient to control blood pressure; and a cardiac pacemaker communicated with the applications module to regulate right atrial pacing for the purpose of controlling blood pressure.

The pacemaker determines if a cardiac paced beat is required and the pacemaker applies supra-threshold pacing to the heart and causes a neuro-inhibitory feedback to lower blood pressure according to a stimulus architecture generated by the applications module, if a cardiac beat is required.

The pacemaker determines if a cardiac paced beat is required and the pacemaker applies sub-threshold pacing to the heart causing a neuro-inhibitory feedback back to lower blood pressure according to a stimulus architecture generated by the applications module, if a cardiac beat is not required.

The applications module includes a PressurePace module for first determining an optimal right atrial pacing rate and includes a Stimulus Architecture Algorithm module (SSA) to calculate the selected stimulus architecture based on the optimal right atrial pacing rate from the PressurePace module.

The applications module constructs an algorithm that uses the measured blood pressure reading to calculate a selected stimulus architecture algorithm for regulating right atrial pacing using artificial intelligence using the measured blood pressure reading.

The applications module constructs an algorithm that uses the measured blood pressure reading to calculate a selected stimulus architecture algorithm for regulating right atrial pacing using artificial intelligence using the measured blood pressure reading and at least input selected from the group of patient age, sex, medications, ambient temperature, altitude, patient's instantaneous reports of symptoms, or other parameters deemed relevant by the attending cardiologist.

Right atrial pacing for the purpose of lowering blood pressure is carried out via the multi-electrode pacing electrode, having at least one conventional right atrial pacing electrode and one or more other pacing electrodes arranged and configured to be disposed anatomically proximate to atrial tissue proximate to sympathetic and/or parasympathetic ganglionic tissues for the purpose of stimulating those ganglionic tissues to lower blood pressure.

Thus, this disclosure further extends to a cardiac pacing lead for stimulating right atrial tissue of the heart and stimulating local sympathetic and/or parasympathetic tissues with the stimulus architecture protocol including: a conventional right atrial pacing lead; and one or more other electrodes arranged and configured to be disposed anatomically proximate to atrial tissue proximate to sympathetic and/or parasympathetic ganglionic tissues for the purpose of stimulating those ganglionic tissues to lower blood pressure.

The conventional right atrial pacing lead and one or more other electrodes deliver a plurality of selected stimulus architectures to the right atrium of a heart which selectively stimulates the right atrial tissue of the heart and selectively stimulates local sympathetic and/or parasympathetic tissues respectively, using an optimized stimulus protocol devised by artificial intelligence for the patient to control blood pressure.

The conventional right atrial electrode delivers a supra-threshold pacing to the heart and at least one of the other electrodes causes a neuro-inhibitory feedback to lower blood pressure according to a stimulus architecture generated by the applications module, if a cardiac beat is required.

The conventional right atrial electrode delivers a sub-threshold pacing to the heart and at least one of the other electrodes causes a neuro-inhibitory feedback to lower blood pressure according to a stimulus architecture generated by the applications module, if a cardiac beat is not required.

A pacing stimulus can be delivered on one electrode without delivery of a pacing stimulus being delivered on other electrodes.

The one or more other electrodes can deliver energy with a specific protocol to the right atrial tissue affecting blood pressure without inducing cardiac pacing.

The illustrated embodiments also extend to a closed loop system including: a skin sensor for real-time measurement of an ANS function to measure skin sympathetic or parasympathetic activity; an applications module receiving the real-time measurement of the ANS function, where the applications module operates under an algorithm that uses the real-time measurement of an ANS function to calculate a selected stimulus architecture of which selectively stimulates the fight atrial tissue of the heart and selectively stimulates local sympathetic and/or parasympathetic tissues using an optimized stimulus protocol to control blood pressure; and a cardiac pacemaker communicated with the applications module to regulate right atrial pacing for the purpose of controlling blood pressure.

The applications module operates under an algorithm by using artificial intelligence to stimulate the right atrial tissue of the heart with the optimal stimulus protocol to control blood pressure.

The closed loop system further includes a blood pressure sensor, the blood pressure sensor and skin sensor communicated with the applications module,

The closed loop system in one embodiment is combined with a smartphone, and the skin sensor is communicated with the applications module through the smartphone.

The disclosure can be better visualized by turning now to the following drawings wherein like elements are referenced by like numerals.

### Brief Description of the Drawings

Fig. 1 is a block diagram illustrating the main elements of an embodiment of the invention.
Fig. 2 is a flow diagram illustrating one embodiment of the methodology of the invention.
Fig. 3 is a simplified diagram of one embodiment of a cardiac lead used in the illustrated embodiment of the invention.
Fig. 4 is a diagram illustrating the SKNA sensing watch or bracelet with a blood pressure sensor communicated to the pacemaker with the possible intermediation of the PressurePace algorithm app and artificial intelligence processing.

The disclosure and its various embodiments can now be better understood by turning to the following detailed description of the preferred embodiments which are presented as illustrated examples of the embodiments defined in the claims. It is expressly understood that the embodiments as defined by the claims may be broader than the illustrated embodiments described below.

### Detailed Description of the Preferred Embodiments

This application expands on the use of either subthreshold and/or non-propagated supra-threshold pacing algorithms in the right atrium designed to affect blood pressure via interaction with epicardial cardiac ganglia or other neuroendocrine mechanisms by means of atrial natriuretic peptides by delivering the pacing impulse to the right atrial tissue. The pacing impulse activates the local sympathetic and/or parasympathetic ganglia producing a neural feedback that results in suppression of blood pressure. A supra-threshold pacing algorithm means the pacing stimuli cause a paced beat unless placed somewhere outside the vulnerable zone of tissue. The pacing stimuli could also be a train of stimuli, none of which is individually supra-threshold, but in aggregate are supra-threshold in total voltage.

It cannot currently be determined as a certainty whether we are stimulating sympathetic, parasympathetic nerve endings, an interplay between the two, or none of the above. Both sympathetic and parasympathetic nerve endings are found in cardiac ganglia. Therefore, where sympathetic tissue is referenced, it is to be understood throughout t this specification that both sympathetic and/or parasympathetic nerve activity may be present, possibly by affecting epicardial ganglionated plexi. In particular, it must be understood that both sympathetic and parasympathetic inputs to the heart are provided.

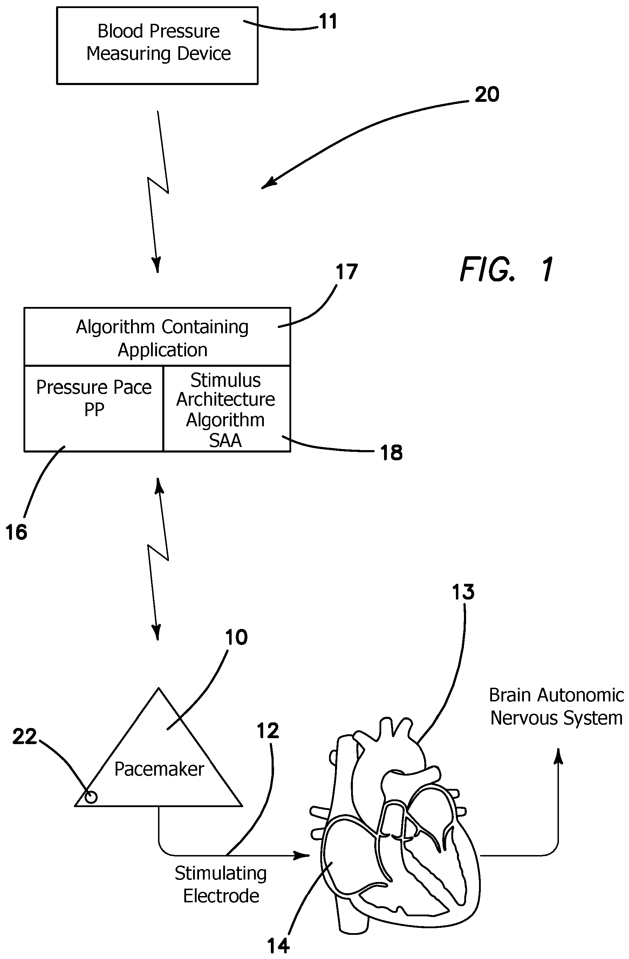

The illustrated embodiment of the invention as diagrammatically depicted in Fig. 1 includes: (a) a pacemaker 10 with a pacing electrode 12 implanted in the right atrium 14 of heart 13, and (b) an algorithmic module 17 that determines the (c) stimulus architecture or protocol delivered through the pacing electrode 12 into the right atrial tissue 14. In one embodiment, the pacing electrode 12 is a conventional right atrial pacing electrode. After receiving the blood pressure reading wirelessly transmitted from blood pressure sensor 11, the PressurePace algorithm or module 16 calculates the optimal right atrial pacing rate as more fully described in the incorporated patent applications. The optimal right atrial pacing rate is then further processed by the Stimulus Architecture Algorithm (SAA) module 18 to arrive at the optimal stimulus protocol or format. The optimal stimulus protocol determines the composite amplitude of the pacing stimulus, the number of sub-pulses within the pacing stimulus totaling the composite delivered amplitude, the frequency of the sub-pulses, the duration of the sub-pulses, and the refractory period before another impulse can be generated.

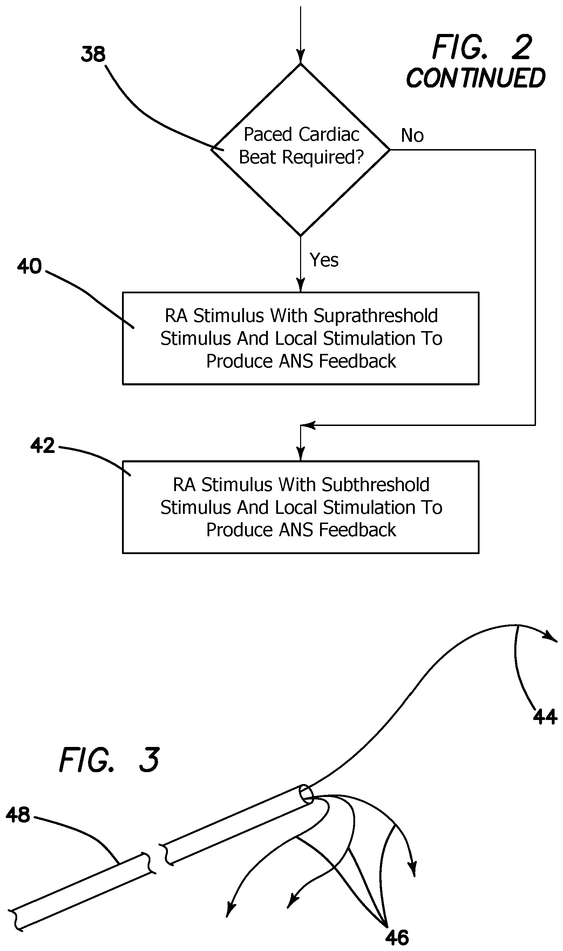

A schematic diagram of the illustrated embodiment of the invention is presented in the diagram of Fig. 1. The components of the system 20 can vary, but essential elements include the pacing electrode12. In other embodiments of the invention the pacing electrode 12 may comprise a plurality of electrodes, which include deliver stimulating pulses to more than one site, where the second or multiple stimulation sites, which can be single or an array, are designed to be positioned closer to the local sympathetic and/or parasympathetic ganglia to more optimally stimulate those autonomic nervous system tissues to produce a neuro-inhibitory effect on blood pressure. Fig. 3 is a simplified diagram of one embodiment of such a multiple electrode lead 48, including a conventional right atrial cardiac lead 44 and a plurality of other neuro-inhibitory leads 46.

System 20 further includes the SAA algorithm or module 18. The SAA algorithm or module 18 uses computational and artificial intelligence analysis methods to determine the optimal stimulus protocol. The optimal stimulus architecture is computed by the SAA algorithm or module 18 to optimally regulate the neuro-inhibitory component of the right atrial pacing effect, taking into account other simultaneous factors including pacemaker rate modulation sensor outputs, e.g. from accelerometer and respiratory rate sensor 22 included in pacemaker 10, and the PressurePace-derived optimal right atrial pacing rate. In alternative embodiments of the PressurePace algorithm or module 16, this will include additional factors previously described in the incorporated patent applications, including but not limited to patient age, sex, medications, ambient temperature, altitude, patient's instantaneous reports of symptoms, and any other parameters deemed relevant by the attending cardiologist.

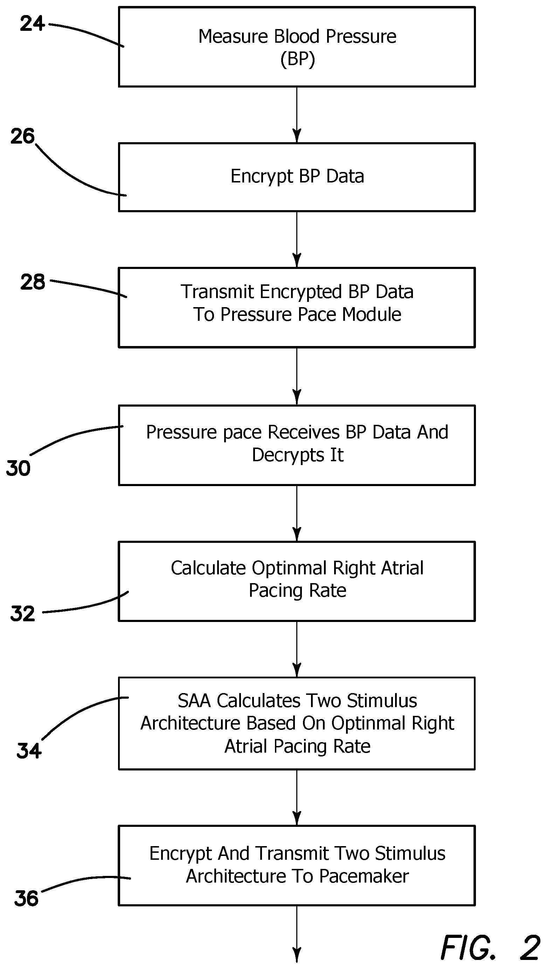

The illustrated embodiment of the methodology is better understood by turning the flow diagram of Fig. 2. At step 24 the patient's blood pressure (BP) is measured using a blood pressure measuring device, The measured blood pressure data is then encrypted at step 26. The encrypted BP data is wirelessly transmit data to the PressurePace software module 16 at step 28. The PressurePace software module 16 receives data and decrypts it at step 30. At step 32 the PressurePace module 16 calculates an optimal right atrial pacing rate, based on a determination made by the corresponding algorithm using artificial intelligence (AI) as set out in the incorporated patent applications.

Computer science defines AI research as the study of "intelligent agents": any device that perceives its environment and takes actions that maximize its chance of successfully achieving its goals. A more elaborate definition characterizes AI as "a system's ability to correctly interpret external data, to learn from such data, and to use those learnings to achieve specific goals and tasks through flexible adaptation. A typical At analyzes its input data and takes actions that maximize its chance of success, in this case reduction of blood pressure. AI often revolves around the use of algorithms. An algorithm is a set of unambiguous instructions that a mechanical computer can execute. A complex algorithm is often built on top of other, simpler, algorithms. Many AI algorithms are capable of learning from data; they can enhance themselves by learning new heuristics (strategies, or "rules of thumb", that have worked well in the past), or can themselves write other algorithms, Some of the "learners" described below, including Bayesian networks, decision trees, and nearest-neighbor, could theoretically, (given infinite data, time, and memory) learn to approximate any function, including which combination of mathematical functions would best describe the world These learners could therefore derive all possible knowledge, by considering every possible hypothesis and matching them against the data. In practice, it is seldom possible to consider every possibility, because of the phenomenon of "combinatorial explosion", where the time needed to solve a problem grows exponentially. Much of AI research involves figuring out how to identify and avoid considering a broad range of possibilities unlikely to be beiieficial.

The earliest (and easiest to understand) approach to AI was symbolism (such as formal logic): "If an otherwise healthy adult has a fever, then they may have influenza". A second, more general, approach is Bayesian inference: "If the current patient has a fever, adjust the probability they have influenza in such-and-such way". The third major approach, extremely popular in routine business AI applications, are analogizers such as support vector machine model (SVM) and nearest-neighbor: "After examining the records of known past patients whose temperature, symptoms, age, and other factors mostly match the current patient, X% of those patients turned out to have influenza". A fourth approach is harder to intuitively understand, but is inspired by how the brain's machinery works: the artificial neural network approach uses artificial "neurons" that can learn by comparing itself to the desired output and altering the strengths of the connections between its internal neurons to "reinforce" connections that seemed to be useful. These four main approaches can overlap with each other and with evolutionary systems; for example, neural nets can learn to make inferences, to generalize, and to make analogies. Some systems implicitly or explicitly use multiple of these approaches, alongside many other AI and non-AI algorithms; the best approach is often different depending on the problem.

Learning algorithms work on the basis that strategies, algorithms, and inferences that worked well in the past are likely to continue working well in the future. These inferences can be obvious, such as "since the sun rose every morning for the last 10,000 days, it will probably rise tomorrow morning as well". They can be nuanced, such as "X% of families have geographically separate species with color variants, so there is a Y% chance that undiscovered black swans exist". Learners also work on the basis of "Occam's razor": The simplest theory that explains the data is the likeliest. Therefore, according to Occam's razor principle, a learner must be designed such that it prefers simpler theories to complex theories, except in cases where the complex theory is proven substantially better.

Settling on a bad, overly complex theory gerrymandered to fit all the past training data is known as overfitting. Many systems attempt to reduce overfitting by rewarding a theory in accordance with how well it fits the data, but penalizing the theory in accordance with how complex the theory is.^{[72]} Besides classic overfitting, learners can also disappoint by "learning the wrong lesson". A toy example is that an image classifier trained only on pictures of brown horses and black cats might conclude that all brown patches are likely to be horses. A real-world example is that, unlike humans, current image classifiers don't determine the spatial relationship between components of the picture; instead, they learn abstract patterns of pixels that humans are oblivious to, but that linearly correlate with images of certain types of real objects. Faintly superimposing such a pattern on a legitimate image results in an "adversarial" image that the system misclassifies.

Stimulus architecture algorithm module (SAA) 18 calculates two different possible stimulus architectures at step 34. The first stimulus architecture includes a supra-threshold stimulus (cardiac pacing is required), which produces both cardiac pacing and stimulation of local sympathetic and/or parasympathetic tissues to produce feedback inhibition of the autonomic nervous system to lower blood pressure. The second stimulus architecture includes sub-threshold stimulus (cardiac pacing is not required) which produces only feedback inhibition of the autonomic nervous system to lower blood pressure.

The two stimulus architectures are encrypted and transmitted at step 36 wirelessly to a pacemaker 10 which is "paired" to receive and decrypt the signal only from stimulus architecture algorithm module (SAA) 18. Then at step 38 pacemaker 10 receives and decrypts the two stimulus architectures.

If the pacemaker programming determines at step 38 that a paced cardiac beat is indicated per standard pacemaker protocol, right atrial stimulation proceeds according to the first of the stimulus architectures at step 40, namely a supra-threshold stimulus (cardiac pacing is required), which produces both cardiac pacing and stimulation of local sympathetic and/or parasympathetic tissues to produce feedback inhibition of the autonomic nervous system to lower blood pressure. If the pacemaker programming determines that a paced cardiac beat is not required at step 38, fight atrial stimulation proceeds at step 42 according to the second of the stimulus architectures, namely sub-threshold stimulus (cardiac pacing is not required) which produces only feedback inhibition of the autonomic nervous system to lower blood pressure.

We have previously disclosed in the incorporated applications the relationship between right atrial (RA) pacing and the treatment of dialysis-related hypotension (DRH) and diastolic heart failure (DHF). We have further described an automatic closed-loop system controlled by an algorithm, PressurePace (PP), comprised of a blood pressure measuring device connected via Bluetooth to a processor containing the PP algorithm such as a smartphone with Bluetooth, that is in turn connected via Bluetooth to a pacemaker to regulate RA pacing. We have suggested a mechanism of action for that system and a mechanical design thereto that involves direct stimulation of the autonomic nervous system (ANS) that does not require the provocation of a paced beat, so called subthreshold pacing controlled by a second algorithm we have called Stimulus Architecture Algorithm (SAA),

Also disclosed is an embodiment, which is an extension of the system described above and which integrates the real-time measurement of ANS function by adding a noninvasive skin sensor to the loop that measures skin sympathetic or parasympathetic activity. In one embodiment the ANS skin sensor is disposed on the back of a smartwatch worn in contact with the patient's skin. The smartwatch is communicated with a smartphone or a computer either by a cable or Bluetooth connection. Turn now and consider the measurement of skin sympathetic or parasympathetic activity (SKNA). SKNA directly and non-invasively records sympathetic or parasympathetic nerve activity. It can be used to measure sympathetic or parasympathetic tone in healthy subjects and in subjects with non-cardiovascular diseases. The electrical activity that can be measured on the surface of the skin originates from the heart, the muscle or nerve structures. Because the frequency content of nerve activity falls in a higher frequency range than that of the ECG and myopotentials, it is necessary to use high-pass or band-pass filtering to specifically isolate the SKNA signal. SKNA is voltage calibrated and does not require invasive procedures to invasively dispose electrodes in nerves and thus has advantages over microneurography. The measurement of SKNA can be performed using commercially available hardware and software.

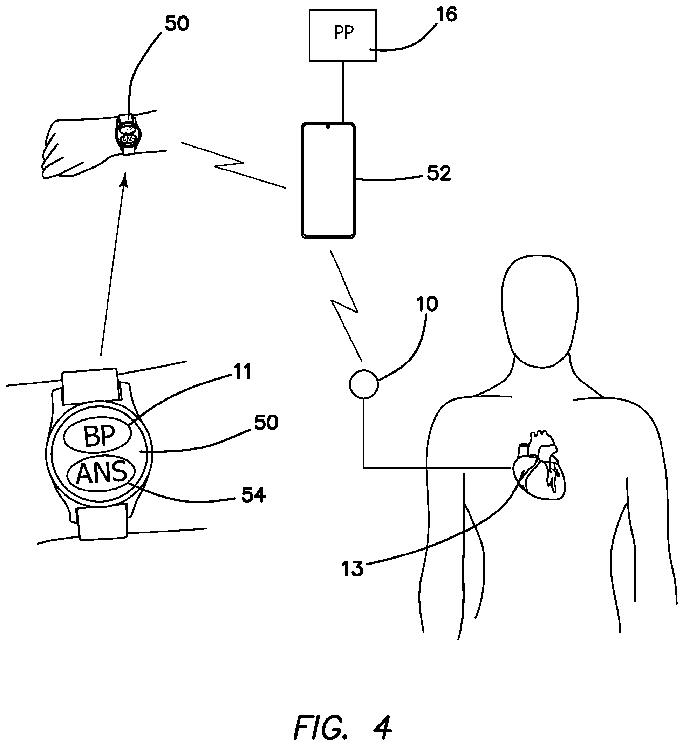

An electrode (sensor) that can receive the SKNA signal is affixed or otherwise affixed to the bottom surface of a non-pneumatic driven blood pressure sensing watch 50 as is currently available and FDA approved diagrammatically illustrated in Fig. 4 This configuration places the electrode in contact with the wearer's skin when the watch is worn on the wrist or other suitable body part. The watch 50 is Bluetooth enabled and configured to communicate with the PressurePace app 16. The watch 50 also contains electronic elements that process the skin electrode's signal as previously described and validated. The same electrode may also be used to receive the patient's ECG for other applications, including other versions of PressurePace algorithm that may include an ECG signal as part of the dataset.

In the preferred embodiment, the watch 50 encrypts both the blood pressure result and the skin sensor output and sends those results to the PressurePace application 16 which is resident in a smart phone 52 that has been paired with watch 50 to receive the processed SKNA signals. As previously described, the PressurePace app 16 decrypts both signals and calculates the optimal RA pacing rate to both lower blood pressure and, in the present embodiment, optimize the sympathetic tone, peripheral resistance or systemic vascular resistance as a secondary factor in regulating blood pressure. The resultant RA pacing command is then encrypted and sent via Bluetooth to the patient's pacemaker 10 which is capable of receiving the signal It is decrypted and the command executed, thereby lowering blood pressure to the most efficient level, and also benefiting the treatment of heart failure with preserved ejection fraction (HFpEF). The process repeats every three minutes, or as otherwise programmed by the supervising physician.

Other embodiments include a version of blood pressure watch 50, which contains the blood pressure sensing sensor 11, the autonomous nervous system fANS) sensor or electrode 54, and the PP app 16. Watch 50 may also include a separate ECG sensing electrode with the AN S sensor or electrode 54 and the PP app 16.
i. In another embodiment the blood pressure measuring device is a conventional pneumatic cuff with Bluetooth capability, and the ANS sensor or electrode 54 is separate and worn separately on the wrist with Bluetooth capability to be sent to the app 16 which is in a smart phone 52. The ANS sensor or electrode 54 is optionally connected to the smartphone 52 via a wire cable that transmits the data. It must be understood that watch 50 may be provided with a variety different kinds of sensing mechanisms and combinations with smartphone 52 and PP app 16 with or without artificial intelligence processing described above. For example, the blood pressure sensor may or may include a manual or automatic pneumatic cuff or may be electronic, may or may not be Bluetooth communicated to the smartphone 52, may or may not be an implanted blood pressure sensor, may or may not have an ANS sensor 54, may be carried separately in a bracelet with or without Bluetooth communication or hardwired cable communication to smartphone 52, may or may not have PP app 16 resident in watch 50, in other blood pressure devices, in smartphone 52, in a separate processing module, or in pacemaker 10. Thus, it is clear it is entirely within the scope of the invention that the elements including watch 50 or a substitute bracelet (not shown), smartphone 52, ANS sensor 54, PP app 16, and pacemaker 10 may be combined in a large number of different equivalent permutations and intercommunicated by Bluetooth or cable with or without artificial intelligence processing.

Many variations and modifications may be made by those having ordinary skill in the art without departing from the scope of the embodiments. Therefore, it must be understood that the illustrated embodiment has been set forth only for the purposes of example and that it should not be taken as limiting the embodiments as defined by the following embodiments and its various embodiments.

Therefore, it must be understood that the illustrated embodiment has been set forth only for the purposes of example and that it should not be taken as limiting the embodiments as defined by the following claims. For example, notwithstanding the fact that the elements of a claim are set forth below in a certain combination, it must be expressly understood that the embodiments includes other combinations of fewer, more or different elements, which are disclosed in above even when not initially claimed in such combinations. A teaching that two elements are combined in a claimed combination is further to be understood as also allowing for a claimed combination in which the two elements are not combined with each other, but may be used alone or combined in other combinations. The excision of any disclosed element of the embodiments is explicitly contemplated as within the scope of the embodiments.

The words used in this specification to describe the various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

## Claims

1. A smart phone device (52), comprising at least one processor and at least one memory comprising instructions that, when executed by the processor, cause the device to:
receive a blood pressure value (30);
calculate a pacing rate (32) based on the blood pressure value (30);
determine a supra-threshold stimulus protocol (34) to produce cardiac pacing and stimulation of one or both of local sympathetic tissue or parasympathetic tissues to modify the blood pressure value, based on the pacing rate; and
determine a sub-threshold stimulus protocol (34) to produce feedback inhibition of a portion of an autonomic nervous system to modify the blood pressure value, based on the pacing rate (32), wherein each of the supra-threshold stimulus protocol (34) and the sub-threshold stimulus protocol (34) comprises a respective composite amplitude of pacing stimulus, frequency of sub-pulses, duration of sub-pulses, and refractory period between a current pacing stimulus and a subsequent pacing stimulus.

2. The smart phone device (52) of claim 1, wherein the respective composite amplitude of pacing stimulus is reached based on a number of sub-pulses, the number of sub-pulses having amplitudes totaling the composite amplitude of pacing stimulus.

3. The smart phone device (52) of any of claims 1-2, wherein the instructions further cause the device to cause a pacing device (10) to apply one of the supra-threshold stimulus protocol (34) based on determining that a paced cardiac beat is indicated or the sub-threshold stimulus protocol (34) based on determining that a paced cardiac beat is not indicated.

4. The smart phone device (52) of any of claims 1-3, wherein the instructions cause the device to calculate the pacing rate by:
comparing the blood pressure value (30) to a target blood pressure value; and
determining the pacing rate based on comparing the blood pressure value to the target blood pressure value.

5. The smart phone device (52) of claim 4, wherein the target blood pressure value is based on one or more of a patient age, a patient sex, a patient medication, an ambient temperature, an altitude, or a patient symptom.

6. The smart phone device (52) of any of claims 1-5, wherein the instructions further cause the device to:
receive a skin sympathetic or parasympathetic activity, SKNA; and
calculate the pacing rate further based on the SKNA.

7. The smart phone device (52) of claim 6, wherein the SKNA is received from at least one of a high-pass or a band-pass filter.

8. The smart phone device (52) of any of claims 1-7, wherein the instructions further cause the device to:
receive autonomic nervous system, ANS, activity from one of a direct sensor or an indirect function; and calculate the pacing rate further based on the ANS activity.

9. The smart phone device (52) of claim 8, wherein the direct sensor is a microneurography sensor.

10. The smart phone device (52) of claim 8, wherein the indirect sensor is a non-invasive sensor.

11. The smart phone device (52) of any of claims 1-10, wherein one or both of the supra-threshold stimulus protocol (34) or the sub-threshold stimulus protocol (34) are output by an artificial intelligence, AI, model.

12. The smart phone device (52) of claim 11, wherein the AI model is trained to output a stimulus protocol based on training data comprising the blood pressure value (30) and one or more of ANS activity, a patient age, a patient sex, a patient medication, an ambient temperature, or a patient symptom.

13. The smart phone device (52) of any of claims 11-12, wherein the AI model is part of an algorithmic module (17) configured to provide the pacing rate to a pacing module.

14. The smart phone device (52) of any of claims 11-13, wherein the pacing module is configured to output at least one of the supra-threshold stimulus protocol or the sub-threshold stimulus protocol to the pacing device (10).

## Patentansprüche

1. Smartphone-Vorrichtung (52) mit mindestens einem Prozessor und mindestens einem Speicher, der Befehle enthält, die, wenn sie von dem Prozessor ausgeführt werden, die Vorrichtung veranlassen zum:
Empfangen eines Blutdruckwerts (30);
Berechnen einer Pacing-Rate (32) auf der Grundlage des Blutdruckwerts (30);
Bestimmen eines Protokolls für überschwellige Stimulation (34) zum Erzeugen eines kardialen Pacings sowie einer Stimulation von lokalem sympathischem Gewebe und/oder parasympathischem Gewebe, um den Blutdruckwert auf der Grundlage der Pacing-Rate zu verändern; und
Bestimmen eines Protokolls für unterschwellige Stimulation (34) zum Erzeugen einer Feedback-Inhibition eines Teils des autonomen Nervensystems, um den Blutdruckwert auf der Grundlage der Pacing-Rate (32) zu modifizieren, wobei sowohl das Protokoll für überschwellige Stimulation (34) als auch das Protokoll für unterschwellige Stimulation (34) eine jeweilige zusammengesetzte Amplitude des Pacing-Stimulus, eine Frequenz von Teilimpulsen, eine Dauer von Teilimpulsen und eine Refraktärzeit zwischen einem aktuellen Pacing-Stimulus und einem nachfolgenden Pacing-Stimulus umfassen.

2. Smartphone-Vorrichtung (52) nach Anspruch 1, wobei die jeweilige zusammengesetzte Amplitude des Pacing-Stimulus basierend auf einer Anzahl von Teilimpulsen erreicht wird, wobei die Anzahl von Teilimpulsen Amplituden aufweist, die in der Summe die zusammengesetzte Amplitude des Pacing-Stimulus ergeben.

3. Smartphone-Vorrichtung (52) nach einem der Ansprüche 1 bis 2, wobei die Befehle die Vorrichtung ferner veranlassen, eine Pacing-Vorrichtung (10) dazu zu veranlassen, entweder das Protokoll für überschwellige Stimulation (34) anzuwenden, basierend auf dem Bestimmen, dass ein stimulierter Herzschlag indiziert ist, oder das Protokoll für unterschwellige Stimulation (34) anzuwenden, basierend auf dem Bestimmen, dass ein stimulierter Herzschlag nicht indiziert ist.

4. Smartphone-Vorrichtung (52) nach einem der Ansprüche 1 bis 3, wobei die Befehle die Vorrichtung veranlassen, die Pacing-Rate zu berechnen, durch:
Vergleichen des Blutdruckwerts (30) mit einem Ziel-Blutdruckwert; und
Bestimmen der Pacing-Rate basierend auf dem Vergleichen des Blutdruckwerts mit dem Ziel-Blutdruckwert.

5. Smartphone-Vorrichtung (52) nach Anspruch 4, wobei der Ziel-Blutdruckwert auf einem oder mehreren von einem Patientenalter, einem Patientengeschlecht, einer Patientenmedikation, einer Umgebungstemperatur, einer Höhe über dem Meeresspiegel oder einem Symptom eines Patienten basiert.

6. Smartphone-Vorrichtung (52) nach einem der Ansprüche 1 bis 5, wobei die Befehle die Vorrichtung ferner veranlassen zum:
Empfangen einer sympathischen oder parasympathischen Hautaktivität (SKNA); und
Berechnen der Pacing-Rate ferner basierend auf der SKNA.

7. Smartphone-Vorrichtung (52) nach Anspruch 6, wobei die SKNA von einem Hochpass- und/oder einem Bandpassfilter empfangen wird.

8. Smartphone-Vorrichtung (52) nach einem der Ansprüche 1 bis 7, wobei die Befehle die Vorrichtung ferner veranlassen zum:
Empfangen einer Aktivität des autonomen Nervensystems (ANS) von einem direkten Sensor oder einer indirekten Funktion; und Berechnen der Pacing-Rate ferner basierend auf der ANS-Aktivität.

9. Smartphone-Vorrichtung (52) nach Anspruch 8, wobei der direkte Sensor ein Mikroneurographie-Sensor ist.

10. Smartphone-Vorrichtung (52) nach Anspruch 8, wobei der indirekte Sensor ein nichtinvasiver Sensor ist.

11. Smartphone-Vorrichtung (52) nach einem der Ansprüche 1 bis 10, wobei das Protokoll für überschwellige Stimulation (34) und/oder das Protokoll für unterschwellige Stimulation (34) von einem Künstliche Intelligenz (KI)-Modell ausgegeben werden.

12. Smartphone-Vorrichtung (52) nach Anspruch 11**,** wobei das Kl-Modell darauf trainiert ist, ein Stimulusprotokoll basierend auf Trainingsdaten auszugeben, die den Blutdruckwert (30) und eines oder mehrere von einer ANS-Aktivität, einem Patientenalter, einem Patientengeschlecht, einer Patientenmedikation, einer Umgebungstemperatur oder einem Symptom eines Patienten umfassen.

13. Smartphone-Vorrichtung (52) nach einem der Ansprüche 11 bis 12, wobei das Kl-Modell Teil eines algorithmischen Moduls (17) ist, das so konfiguriert ist, dass es die Pacing-Rate an ein Pacing-Modul bereitstellt.

14. Smartphone-Vorrichtung (52) nach einem der Ansprüche 11 bis 13, wobei das Pacing-Modul so konfiguriert ist, dass es ein Protokoll für überschwellige Stimulation und/oder ein Protokoll für unterschwellige Stimulation an die Pacing-Vorrichtung (10) ausgibt.

## Revendications

1. Dispositif de téléphone intelligent (52) comprenant au moins un processeur et au moins une mémoire comportant des instructions qui, quand elles sont exécutées par le processeur, commandent au dispositif de :
recevoir une valeur de pression artérielle (30) ;
calculer une fréquence d'excitation (32) sur la base de la valeur de pression artérielle (30) ;
déterminer un protocole de stimulus supraliminaire (34) pour produire une excitation cardiaque et une stimulation d'un tissu sympathique et/ou parasympathique local afin de modifier la valeur de pression artérielle, sur la base de la fréquence d'excitation ; et
déterminer un protocole de stimulus infraliminaire (34) pour produire une inhibition par rétroaction d'une portion d'un système nerveux autonome afin de modifier la valeur de pression artérielle, sur la base de la fréquence d'excitation (32), dans lequel chaque protocole parmi le protocole de stimulus supraliminaire (34) et le protocole de stimulus infraliminaire (34) comprend une amplitude composite respective de stimulus d'excitation, une fréquence des sous-impulsions, une durée des sous-impulsions, et une période réfractaire entre un stimulus d'excitation actuel et un stimulus d'excitation subséquent.

2. Dispositif de téléphone intelligent (52) selon la revendication 1, dans lequel l'amplitude composite respective du stimulus d'excitation est atteinte sur la base d'un nombre de sous-impulsions, le nombre de sous-impulsions ayant des amplitudes totalisant l'amplitude composite du stimulus d'excitation.

3. Dispositif de téléphone intelligent (52) selon l'une quelconque des revendications 1 et 2, dans lequel les instructions commandent en outre au dispositif de commander à un dispositif d'excitation (10) d'appliquer un protocole parmi le protocole de stimulus supraliminaire (34) basé sur la détermination du fait qu'un battement cardiaque excité est indiqué, et le protocole de stimulus infraliminaire (34) basé sur la détermination du fait qu'un battement cardiaque excité n'est pas indiqué.

4. Dispositif de téléphone intelligent (52) selon l'une quelconque des revendications 1 à 3, dans lequel les instructions commandent au dispositif de calculer la fréquence d'excitation par :
une comparaison de la valeur de pression artérielle (30) à une valeur de pression artérielle cible ; et
une détermination de la fréquence d'excitation sur la base d'une comparaison de la valeur de pression artérielle à la valeur de pression artérielle cible.

5. Dispositif de téléphone intelligent (52) selon la revendication 4, dans lequel la valeur de pression artérielle cible est basée sur un ou plusieurs paramètres parmi l'âge du patient, le sexe du patient, les médicaments du patient, la température ambiante, l'altitude, et un symptôme du patient.

6. Dispositif de téléphone intelligent (52) selon l'une quelconque des revendications 1 à 5, dans lequel les instructions commandent en outre au dispositif de :
recevoir une activité cutanée sympathique ou parasympathique, SKNA ; et
calculer la fréquence d'excitation sur la base en outre du SKNA.

7. Dispositif de téléphone intelligent (52) selon la revendication 6, dans lequel le SKNA est reçu d'au moins un filtre parmi un filtre passe-haut et un filtre passe-bande.

8. Dispositif de téléphone intelligent (52) selon l'une quelconque des revendications 1 à 7, dans lequel les instructions commandent en outre au dispositif de :
recevoir l'activité du système nerveux autonome, SNA, provenant d'une source parmi un capteur direct et une fonction indirecte ; et
calculer la fréquence d'excitation sur la base en outre de l'activité du SNA.

9. Dispositif de téléphone intelligent (52) selon la revendication 8, dans lequel le capteur direct est un capteur de microneurographie.

10. Dispositif de téléphone intelligent (52) selon la revendication 8, dans lequel le capteur indirect est un capteur non invasif.

11. Dispositif de téléphone intelligent (52) selon l'une quelconque des revendications 1 à 10, dans lequel le protocole de stimulus supraliminaire (34) et/ou le protocole de stimulus infraliminaire (34) sont produits par un modèle d'intelligence artificielle, IA.

12. Dispositif de téléphone intelligent (52) selon la revendication 11, dans lequel le modèle d'IA est entraîné à produire un protocole de stimulus sur la base de données d'entraînement comprenant la valeur de pression artérielle (30) et un ou plusieurs paramètres parmi l'activité du SNA, l'âge du patient, le sexe du patient, les médicaments du patient, la température ambiante, et un symptôme du patient.

13. Dispositif de téléphone intelligent (52) selon l'une quelconque des revendications 11 et 12, dans lequel le modèle d'IA fait partie d'un module algorithmique (17) configuré pour fournir la fréquence d'excitation à un module d'excitation.

14. Dispositif de téléphone intelligent (52) selon l'une quelconque des revendications 11 à 13, dans lequel le module de stimulation est configuré pour envoyer au moins un protocole parmi le protocole de stimulus supraliminaire et le protocole de stimulus infraliminaire au dispositif d'excitation (10).
